Europäisches Patentamt

European Patent Office

Office européen des brevets

(11) Publication number: **0 379 581 B1**

# EUROPEAN PATENT SPECIFICATION

(45) Date of publication of patent specification: **10.08.94**   (51) Int. Cl.⁵: **D06M 11/00, D06M 15/03, D06M 23/00, D21H 21/36**

(21) Application number: **89905193.2**

(22) Date of filing: **27.04.89**

(86) International application number: **PCT/JP89/00443**

(87) International publication number: **WO 89/12713 (28.12.89 89/30)**

(54) **DEODORANT MATERIAL AND PROCESS FOR ITS PRODUCTION.**

(30) Priority: **23.06.88 JP 153471/88**
**01.07.88 JP 162476/88**
**18.10.88 JP 260435/88**

(43) Date of publication of application:
**01.08.90 Bulletin 90/31**

(45) Publication of the grant of the patent:
**10.08.94 Bulletin 94/32**

(84) Designated Contracting States:
**BE CH DE FR GB IT LI**

(56) References cited:
**FR-A- 974 173**      **FR-A- 1 122 309**
**GB-A- 598 556**      **JP-A-54 160 900**
**JP-A-62 238 866**     **JP-A-62 238 866**
**JP-B- 5 322 555**

(73) Proprietor: **Kohjin Co., Ltd.**
**No. 1-1, Shinbashi 1 chome**
**Minato-ku Tokyo 105 (JP)**

(72) Inventor: **YAMAJI, Keizou c/o Kohjin Pulp Co., Ltd**
**11277 Higashihama, Saiki City**
**Ooita 876 (JP)**
Inventor: **SATOU, Natumi c/o Kohjin Pulp Co., Ltd.**
**11277 Higashima, Saiki City**
**Ooita 876 (JP)**
Inventor: **MORISAKI, Eiji c/o Kohjin Pulp Co., Ltd.**
**11277 Higashihama, Saiki City**
**Ooita 876 (JP)**

(74) Representative: **Patentanwälte Wenzel & Kalkoff**
**Postfach 24 48**
**D-58414 Witten (DE)**

**Description**

The present invention relates to a deodorizing material useful for the removal of malodorous substances which are present in rooms, refrigerators, etc. or under various environment and to a process for producing the same.

As deodorizing materials for removing malodorous substances, such as ammonia, methyl mercaptan, methyl sulfide, dimethyl disulfide, hydrogen sulfide and trimethylamine, which are present in rooms, refrigerators, etc., there have hitherto been proposed those consisting mainly of activated carbon; those consisting of fibers or the like to which phthalocyanine complexes are attached; and those consisting of a carboxymethylated cellulose on which copper and/or zinc ions are adsorbed.

However, deodorants consisting mainly of activated carbon are in the form of granules, and they are colored in black. Therefore, the deodorants are employed in the state contained in a good-looking package. This results in bulkiness and poses various constraints on their use.

Deodorants consisting of fibers, such as celluloses, to which phthalocyanine complexes are attached are slow in the speed of deodorization and hence their deodorizing capability is insufficient from practical point of view.

Deodorants comprising carboxymethylated cellulose fibers on which copper and/or zinc ions are adsorbed could hardly be said to be a practical deodorizer since the ions are adsorbed thereon in only small quantities.

There has also been reported cellulose fibers dipped in a concentrated alkali solution of a copper compound to attain an increase in the quantity of ions adsorbed thereon. However, cellulose fibers so treated are severely damaged by the strong alkali and hence become unsatisfactory in workability, processability and other practical properties,

It has therefore been desired to develop a deodorizing material which is excellent in processability and exhibits excellent deodorizing properties.

With the invention as disclosed in the appending claims the copper hydroxide and/or zinc hydroxide can be effectively attached to and fixed on the cellulose fibers under certain conditions and the fixed fibers so obtained exhibit excellent deodorizing effects against a wide range of malodorous substances.

In JP-A-62-238866 there is disclosed a process for preparing deodorizing fibers by impregnating them with a sodium hydroxide solution containing powders of copper hydroxide or copper oxide. However, the adherence of copper hydroxide in powder form to fibers is inferior to the adherence of copper hydroxide presented in a colloidal solution.

In JP-A-54-160900 a process is disclosed which concerns copper hydroxide dissolved in an alkaline solution containing fibers. Again, the adherence of the copper hydroxide is inferior compared to treating the fibers with a colloidal solution.

As examples of cellulose fibers usable in the present invention, mention may be made of pulp fibers, such as bleached sulfite pulps (e.g., NBSP, LBSP, NDSP, LDSP, etc.) and bleached kraft pulps (e.g., NBKP, LBKP, etc.); flaxes, such as Manila hemp, jute, etc.; cottons, such as cotton wool, cotton linter, etc.; natural fibers of <u>kozo</u> (paper mulberry), <u>mitsumata</u> (<u>Edgeworthis papyrifera</u>), etc. and their pulpy derivatives; rayon; and oxidized cellulose-containing fibers obtainable through oxidation of these fibers. These cellulose fibers can be used either individually or in combination of two or more of them.

In the present invention, deodorizing fibers are produced by fixing copper hydroxide and/or zinc hydroxide on at least one of the above-mentioned cellulose fibers by means of contact with a liquid containing colloids of copper hydroxide and/or zinc hydroxide.

If cellulose fibers are dipped in a dispersion containing copper hydroxide and/or zinc hydroxide in a non-colloidal solid state, the components could hardly be fixed strongly on the fibers, and particles of the components easily drop off during handling. There will therefore be resulted a product which could hardly be a practical deodorizer.

On the other hand, if cellulose fibers are dipped in a solution of copper hydroxide and/or zinc hydroxide in which no colloids are formed, the copper hydroxide and/or zinc hydroxide will attach to the cellulose fibers in only small quantities, and hence there will be resulted a product which is only unsatisfactory as a practical deodorizing material.

Copper hydroxide and/or zinc hydroxide can be attached to and fixed on cellulose fibers in a colloidal state, e.g., in the following manner:

Method for Fixing

To an aqueous solution of a water-soluble copper compound and/or a water-soluble zinc compound in which cellulose fibers are dispersed is added an alkaline substance, so as to form colloid by adjusting its pH to 4.5 to 12 in the case of a copper compound or to 6.2 to 12 in the case of a zinc compound, preferably to 8.0 to 9.5 in either case. Or colloid is formed in the above manner, and then cellulose fibers are charged and dispersed into the colloid-containing solution. Copper hydroxide and/or zinc hydroxide is attached to and fixed on the cellulose fibers by way of contact between the colloid-containing solution and the fibers. Colloidal particles could hardly be formed unless the pH is in the range of 4.5 to 12 in the case of a copper compound or in the range of 6.2 to 12 in the case of a zinc compound. If the fixation is carried out at a pH lower than 8.0, the quantity of fixed compounds and the rate of their fixing will become lower and, on the contrary, if it is effected at a pH higher than 9.5, the cellulose fibers tend to become brittle. The pH range of from 8.0 to 9.5 is therefore preferred. The cellulose fibers, when subjected to the fixation at a relatively high pH, are preferably washed well with water.

In cases, in particular, where the pH is higher than ca. 12, the fibers are severely damaged since they are cellulosic, and after treatment of the resulting fibers, such as washing or the like, must be intensified. In addition, there will be resulted an undesirable lowering in the strength of the fibers and deterioration in their processability.

In the deodorizing fibers of the present invention, the components which contribute to their capability of deodorization are present on the surface of the fibers and come to contact with malodorous gases, the subjects for deodorization, in an effective manner. Because of this, the deodorizing fibers provide an improved deodorizing effect compared with fibers obtained by means of dipping in a simple aqueous solution of a copper compound.

In the above-described preparation of aqueous solution of a water-soluble copper compound and/or a water-soluble zinc compound, the concentration of the components must be within the range where it is possible to form a colloidal solution when the pH is adjusted later.

Cellulose fibers can be subjected to an acid treatment by using, e.g., hydrochloric acid, sulfuric acid, sulfurous acid, nitric acid, etc. before being dispersed into the aqueous solution of a water-soluble copper and/or zinc compound. Alternatively, cellulose fibers can be subjected to a chitosan treatment, dipping them in an aqueous acidic solution of chitosan, so as to attain an enhancement in the quantity of the desired hydroxides attached thereto. This effect is particularly marked when zinc hydroxide is employed.

There are no particular restrictions on water-soluble copper and zinc compounds to be used in the present invention. Any copper and zinc compounds can be used only if they are soluble in water. As examples of such compounds, mention may be made of copper sulfate, copper chloride, copper nitrate, copper acetate, zinc sulfate, zinc chloride, zinc nitrate, zinc acetate, and the like.

As alkaline substances for providing alkalinity, there can be used any compounds which are capable of reacting with the above-described copper and zinc compounds to form colloidal copper hydroxide and/or zinc hydroxide. As examples of such alkaline substances, mention may be made of sodium hydroxide, potassium hydroxide, sodium carbonate, sodium bicarbonate, and the like. Of these compounds, sodium hydroxide and potassium hydroxide can be preferable because of easiness of the pH adjustment.

Deodorizing fibers obtained through the hydroxide-fixing treatment and, where desired, washing with water and drying described hereinbefore can be used as they are as a deodorizing material or can be shaped into a sheet-like product which is made by a conventional paper milling method or a three-dimensional product to be used as a deodorizing material.

Upon the production of shaped products, there can be used more than one kind of cellulose fibers which have copper and/or zinc hydroxide fixed thereon, and it is possible to use the cellulose fibers together with cellulose fibers and/or other fibers not fixed with copper and/or zinc hydroxide, within the limits where the required deodorizing capability can be satisfied. In cases where they are shaped into a sheet-like product, granules, or the like, it is possible to incorporate therein auxiliary agents conventionally used for paper milling, such as wet strength intensifiers, polymeric coagulants, etc., within the limits that the practical deodorizing capability of the shaped product is not impaired.

Furthermore, it is possible to subject the thus obtained shaped products to a secondary processing, such as surface printing, lamination with other materials, folding, and shaping into a three-dimensional form.

Best Mode for Practicing the Invention

The present invention will further be explained in detail by way of examples. However, the invention is by no means limited to these.

Analytical values shown in the examples were determined in the following manner:

(1) Concentration of Cu and Zn

Determined by the atomic absorption photometry.

(2) Relative viscosity

Measured in accordance with JIS P 8101.

(3) Moisture content in samples

Measured in accordance with JIS P 8203.

(4) Deodorizing capability

Into a 1.5 liter polyvinyl chloride bag were charged 1 g of a sample of a deodorant and 1.5 liters of a malodorous gas of a predetermined concentration (100 ppm in each case), and then the bag was sealed. The concentration of the malodorous gas remaining in the bag was measured with a gas detection tube immediately after the sealing and 10, 30 and 60 minutes after the sealing, and the remaining rate (%) of the gas was calculated therefrom.

Examples 1-3

To 20 liters of water was added 1,000 g of NBSP, of bleached sulfite pulps (used as a cellulose fiber), and the mixture was disaggregated to a pulpy state by using a disaggregator and then subjected to an acid treatment by the addition of an aqueous $SO_2$ solution up to a pH of 3.3. Subsequently, an aqueous copper sulfate solution containing 200 g/l of $CuSO_4 \cdot 5H_2O$ was added there to up to a concentration of 3 W/W% (reduced to copper and based on the weight of the NBSP). Then, the pH of the mixture was adjusted to 5.0, 6.0 or 9.5 (Example 1, 2 and 3, respectively) by using an aqueous solution of sodium hydroxide (120 g/l), whereby colloid of copper hydroxide was formed, and the colloid formed was attached to, and fixed on, the NBSP fibers to produce deodorizing fibers. The thus obtained fibers were shaped into sheets by using a sheet machine and then dried to give sheets of ca. 410 g/m$^2$. The quantity of copper hydroxide fixed on each sheet was determined (in W/W% reduced to copper and based on the weight of the NBSP), and the rate(%) of copper fixed, based on the weight of copper added, was calculated therefrom. Results obtained are shown in Table 1.

It would be apparent from the results that excellent fixed quantities and fixed rates could be attained at a pH of 6.0 and above.

EXAMPLES 4-6

To 20 liters of water was added 1,000 g of NBSP (cellulose fiber), and the mixture was disaggregated to a pulpy state by using a disaggregator, and its pH was adjusted to 3.0 by the addition of an aqueous $SO_2$ solution. Subsequently, an aqueous zinc sulfate solution containing 200 g/l of $ZnSO_4$ was added thereto up to a concentration of 3 W/W% (reduced to zinc and based on the weight of the NBSP). Then, the pH of the mixture was adjusted to 6.5, 8.0 or 9.5 (Example 4, 5 or 6, respectively) by using an aqueous solution of sodium hydroxide (120 g/l), whereby colloid of zinc hydroxide was formed, and the colloid formed was attached to, and fixed on, the NBSP fibers to produce deodorizing fibers. The thus obtained fibers were shaped into sheets by using a sheet machine and then dried to give sheets of ca. 410 g/m$^2$. The quantity of zinc hydroxide fixed on each sheet was determined (in W/W% reduced to zinc and based on the weight of the NBSP), and the rate(%) of zinc fixed, based on the weight of zinc added, was calculated therefrom. Results obtained are also shown in Table 1.

It would be apparent from the results that excellent fixed quantities and fixed rates could be attained at a pH of 8.0 and above.

4

EXAMPLES 7-9

To 20 liters of water was added 1,000 g of NBSP, and the mixture was disaggregated to a pulpy state by using a disaggregator and then subjected to an acid treatment by the addition of an aqueous $SO_2$ solution up to a pH of 3.3. Subsequently, an aqueous copper solution containing 200 g/l of $CuSO_4 \cdot 5H_2O$ was added thereto in quantities as shown in Table 1 based on weight reduced to copper (Examples 7, 8 and 9). Then, the pH of the mixtures was adjusted to 8 by the addition of an aqueous solution of sodium hydroxide (120 g/l), whereby colloid of copper hydroxide was formed, and the colloid formed was attached to, and fixed on, the NBSP fibers to produce deodorizing fibers. The thus obtained fibers were shaped into sheets by using a sheet machine and then dried to give sheets of ca. 410 $g/m^2$.

The quantity of copper hydroxide fixed on each of the sheets was determined (in W/W% reduced to copper and based on the weight of the NBSP), and the rate(%) of copper fixed, based on the weight of copper added, was calculated therefrom. Results obtained are shown in Table 2.

Thereafter, deodorizing capability for $H_2S$ gas, $NH_3$ gas and methyl mercaptan gas of the products according to Examples 7, 8 and 9 was evaluated in accordance with the test method shown hereinbefore. Results obtained are also shown in Table 3.

It would be apparent from the results shown in Tables 2 and 3 that the deodorizing materials according to the invention are capable of effectively acting on such malodorous gaseous substances as $H_2S$, $NH_3$ and methyl mercaptan.

Examples 10-11

To 20 liters of water was added 1,000 g of NBSP (cellulose fiber), and the mixture was disaggregated to a pulpy state by using a disaggregator, and its pH was adjusted to 3.0 by the addition of an aqueous $SO_2$ solution. Subsequently, an aqueous zinc sulfate solution was added thereto up to a concentration of 2 or 6 W/W% (reduced to zinc and based on the weight of the NBSP) [Example 10 or 11, respectively]. Then, the pH of the mixtures was adjusted to 8 by the addition of an aqueous solution of sodium hydroxide (120 g/l), whereby colloid of zinc hydroxide was formed, and the colloid formed was attached to, and fixed on, the NBSP fibers to produce deodorizing fibers. The thus obtained fibers were shaped into sheets by using a sheet machine and then dried to give sheets of ca. 410 $g/m^2$.

The quantity of zinc hydroxide fixed on each of the sheets was determined (in W/W% reduced to zinc and based on the weight of the NBSP), and the rate(%) of zinc fixed, based on the weight of zinc added, was calculated therefrom. Results obtained are also shown in Table 2.

Thereafter, the deodorizing capability for $H_2S$ gas and $NH_3$ gas of the products according to Examples 10 and 11 was evaluated in accordance with the the test method described hereinbefore. It would be apparent from the results shown in Table 2 that the cellulose fibers on which zinc hydroxide is fixed possess the capability of deodorizing $H_2S$ gas and $NH_3$ gas.

Example 12

Disaggregated and acid treated slurry of NBSP fibers was prepared in the same manner as in the case of aqueous copper sulfate solution in Example 9, and copper sulfate and zinc sulfate were added thereto in the same manner as in Example 9, up to a concentration of 2% by weight each (reduced to copper or zinc and based on the weight of the fibers). Subsequently, the pH of the mixture was adjusted to 8 by the addition of an aqueous solution of sodium hydroxide (120 g/l), whereby a copper hydroxide and zinc hydroxide-containing colloid was formed, and the colloid formed was attached to, and fixed on, the NBSP fibers to produce deodorizing fibers. The fibers obtained were shaped into a sheet by using a sheet machine and then dried to give a sheet of ca. 410 $g/m^2$.

Thereafter, the deodorizing capability for $H_2S$ gas and $NH_3$ gas of the thus obtained sheet was evaluated in accordance with the test method described hereinbefore. Results obtained are also shown in Table 2.

It would be apparent from the results shown in Table 2 that the deodorizing material according to the invention is capable of effectively acting on such malodorous gaseous substances as $H_2S$ and $NH_3$.

Fixed quantity:

Cu: 1.8% by weight
Zn: 1.3% by weight

EP 0 379 581 B1

Deodorizing capability:

|  | Immediately after sealing | After 10 min. | After 30 min. | After 60 min. |
|---|---|---|---|---|
| $H_2S$ | 30 | 2 | 0 |  |
| $NH_3$ | 19 |  | 0 |  |

Example 13

To 20 liters of water was added 1,000 g of NBSP (oxidized cellulose fiber), and the mixture was disaggregated to a slurry by using a disaggregator. An aqueous 1 wt% chitosan solution in 1 wt% acetic acid was added to the mixture, up to a concentration of chitosan of 1.0% by weight, based on the weight of the NBSP, and the resulting mixture was stirred for 15 minutes. Thereafter, an aqueous zinc sulfate solution was added thereto up to a concentration of zinc of 6 W/W%, based on the weight of the NBSP. Then, the pH of the mixture was adjusted to 8 by the addition of an aqueous solution of sodium hydroxide (120 g/l), whereby colloid of zinc hydroxide was formed, and the colloid formed was added to, and fixed on, the NBSP fibers to produce deodorizing fibers.

The fibers were shaped into a sheet by using a sheet machine and then dried to give a sheet of ca. 410 $g/m^2$.

Then, the quantity of zinc hydroxide fixed on the sheet was determined (in W/W% reduced to zinc and based on the weight of the NBSP), and the rate(%) of zinc fixed, based on the weight of zinc added, was calculated therefrom. Results obtained are also shown in Table 2.

Thereafter, the deodorizing capability for $H_2S$ gas and $NH_3$ gas of the product according to Examples 10-11 was evaluated in accordance with the test method described hereinbefore.

Fixed quantity: 4.9% by weight
Fixed rate: 79%

Deodorizing capability:

|  | Immediately after sealing | After 10 min. | After 30 min. | After 60 min. |
|---|---|---|---|---|
| $H_2S$ | 29 | 2 | 0 |  |
| $NH_3$ | 19 | 5 | 0 |  |

Example 14

To 20 liters of an alkaline solution whose pH had been adjusted to 9.5 by the addition of sodium hydroxide was added 1,000 g of NBSP, and the mixture was disaggregated to a slurry by using a disaggregator. Subsequently, an aqueous copper sulfate solution containing 200 g/l of $CuSO_4 \cdot 5H_2O$ was added thereto up to an amount of copper of 2% by weight, based on the weight of NBSP. Then, the pH of the mixture was adjusted to 8 by using an aqueous solution of sodium hydroxide (120 g/l), whereby colloid of copper hydroxide was formed, and the colloid formed was attached to, and fixed on, the NBSP to produce deodorizing fibers. The fibers were shaped into a sheet by using a sheet machine and then dried to give a sheet of 410 $g/m^2$.

The quantity of copper hydroxide fixed on the sheet was determined (in W/W% reduced to copper and based on the weight of the NBSP), and the rate(%) of copper fixed, based on the weight of zinc added, was calculated therefrom. Results obtained are also shown in Table 2.

6

Comparative Examples 1-2

To 20 liters of water was added 1,000 g of NBSP (cellulose fiber), and the mixture as disaggregated to a slurry by using a disaggregator. Subsequently, a 4 W/W% dispersion of commercially available copper hydroxide powders was added to the slurry of pulp up to a weight ratio of NBSP/Cu = 98/2 or 94/4 (Comparative Example 1 or 2, respectively). After being stirred for 30 minutes, each of the mixtures was shaped into a sheet by using a sheet machine and then dried to give a sheet of ca. 410 g/m$^2$.

Copper hydroxide was not fixed well on any of the sheets, and powders of copper hydroxide dropped off when the sheets were rubbed with a finger.

Comparative Examples 3-4

To 20 liters of water was added 1,000 g of NBSP (cellulose fiber), and the mixture was disaggregated to a slurry by using a disaggregator. Subsequently, a 4 W/W% dispersion of commercially available zinc hydroxide powders was added to the slurry of pulp up to a weight ratio of NBSP/Zn = 98/2 or 94/4 (Comparative Example 3 or 4, respectively). After being stirred for 30 minutes, each of the mixtures was shaped in a sheet by using a sheet machine and then dried to give a sheet of 410 g/m$^2$.

Zinc hydroxide was not fixed well on any of the sheets, and powders of copper hydroxide dropped off when the sheets were rubbed with a finger.

Example 15

1,000 g of NDSP (used as a cellulose fiber) was added to 20 liters of water, disaggregated to the state of slurry by using a disaggregator and then subjected to an acid treatment by the addition of an aqueous $SO_2$ solution up to a pH of 3.3. Subsequently, an aqueous copper chloride solution ($CuCl_2 \cdot 2H_2O$) was added thereto up to a concentration of 6 W/W% (reduced to copper and based on the weight of the NDSP). Then, the pH of the mixture was adjusted to 8 by the addition of an aqueous solution of sodium hydroxide (120 g/l), whereby colloid of copper hydroxide was formed, and the colloid formed was attached to, and fixed on, the NDSP fibers to produce deodorizing fibers.

The fibers were then shaped into a sheet by using a sheet machine and then dried to give a sheet of ca. 410 g/m$^2$.

The quantity of copper hydroxide fixed on the sheet was determined (in W/W% reduced to copper and based on the weight of the NDSP), and the rate(%) of copper fixed thereon, based on the weight of copper added, was calculated therefrom. Results obtained are also shown in Table 2.

Thereafter, the deodorizing capability for $H_2S$ gas and $NH_3$ gas of the sheet was evaluated in accordance with the test method described hereinbefore. Results obtained are shown in Table 2.

It would be apparent from the results shown in Table 2 that the object of the present invention can also be attained in the case where copper chloride is used for the formation of copper hydroxide colloid.

Example 16

1,000 g of cotton wool (used as a cellulose fiber) was added to 20 liters of water, disaggregated to the state of slurry by using a disaggregator and then subjected to an acid treatment by the addition of an aqueous $SO_2$ solution up to a pH of 3.3. Subsequently, an aqueous copper sulfate solution containing 200 g/l of $CuSO_4 \cdot 5H_2O$ was added thereto up to a concentration of 4 W/W% (reduced to copper and based on the weight of the cotton wool). Thereafter, the pH of the mixture was adjusted to 8 by the addition of an aqueous solution of sodium hydroxide (120 g/l), whereby colloid of copper hydroxide was formed, and the colloid formed was attached to, and fixed on, the cotton wool to give deodorizing fibers.

The thus obtained dispersion was shaped into a sheet by using a sheet machine and then dried to give a sheet of ca. 410 g/m$^2$.

The quantity of copper hydroxide fixed on the sheet was determined (in W/W% reduced to copper and based on the weight of the cotton wool), and the rate(%) of copper fixed thereon, based on the weight of copper added, was calculated therefrom. Results obtained are also shown in Table 2.

Thereafter, the deodorizing capability for $H_2S$ gas and $NH_3$ gas of the sheet was evaluated in accordance with the test method described hereinbefore. Results obtained are shown in Table 2.

It would be apparent from the results shown in Table 2 that the object of the present invention can also be attained by using cotton wool.

## Example 17

1,000 g of rayon (used as a cellulose fiber) was added to 20 liters of water, disaggregated to the state of slurry by using a disaggregator and then subjected to an acid treatment by the addition of an aqueous $SO_2$ solution up to a pH of 3.3. Subsequently, an aqueous copper sulfate solution containing 200 g/l of $CuSO_4 \cdot 5H_2O$ was added thereto up to a concentration of 4 W/W% (reduced to copper and based on the weight of the rayon). Thereafter, the pH of the mixture was adjusted to 8 by the addition of an aqueous solution of sodium hydroxide (120 g/l), whereby colloid of copper hydroxide was formed, and the colloid formed was attached to, and fixed on, the rayon fibers to give deodorizing fibers.

The fibers obtained were shaped into a sheet by using a sheet machine and then dried to give a sheet of ca. 410 $g/m^2$.

The quantity of copper hydroxide fixed on the sheet was determined (in W/W% reduced to copper and based on the weight of the rayon), and the rate(%) of copper fixed, based on the weight of the copper added, was calculated therefrom. Results obtained are also shown in Table 2.

Thereafter, deodorizing capability for $H_2S$ gas and $NH_3$ gas of the sheet was evaluated in accordance with the test method described hereinbefore. Results obtained are shown in Table 2.

It would be apparent from the results shown in Table 2 that the objects of the present invention can also be attained by using rayon.

## Example 18

100 g of NBKP (used as a cellulose fiber) was dipped in 300 g of water and then ground by a grinder.

Subsequently, an aqueous $SO_2$ solution was added thereto up to a pH of 3, and after being stirred further, an aqueous copper sulfate solution containing 200 g/l of $CuSO_4 \cdot 5H_2O$ was added thereto up to a concentration of 4 W/W% (reduced to copper and based on the weight of the NBKP). Thereafter, an aqueous solution of sodium hydroxide (120 g/l) was added up to a pH of 8, and the mixture was stirred further to prepare a dispersion. In a separate operation, a dispersion was prepared by disaggregating untreated NBKP to the state of slurry, and then admixed with the dispersion prepared above at a ratio of 1:1, based on the weight of solids. The resulting product was shaped into a sheet by using a sheet machine and dried to give a sheet of ca. 410 $g/m^2$.

The deodorizing capability for $H_2S$ gas and $NH_3$ gas of the sheet was evaluated in accordance with the test method described hereinbefore. Results obtained are shown in Table 2.

## Example 19

A sheet was prepared in the same manner as in Example 16, and 5 g of the sheet was dipped with stirring in 50 ml of diluted aqueous ammonia solution (concentration: 2,000 ppm). After it had been allowed to stand for 1 hour, it was tried to smell the odor of ammonia of the aqueous solution, but no ammonia odor was detected.

## Example 20

A sheet was prepared in the same manner as in Example 16, and 5 g of the sheet was dipped with stirring in 50 ml of diluted aqueous $H_2S$ solution (concentration: 4,000 ppm). After it had been allowed to stand for 1 hour, it was tried to smell the odor of $H_2S$, but no $H_2S$ odor was detected.

## Example 21

1,000 g of cotton wool (cellulose fiber) was added to 20 liters of water and disaggregated to the state of slurry by using a disaggregator, and then an aqueous $SO_2$ solution was added thereto up to a pH of 3.0. Subsequently, an aqueous solution of zinc chloride (200 g/l) was added thereto up to a concentration of 4 W/W% (reduced to zinc and based on the weight of the cotton wool). The pH of the mixture was adjusted to 8 by the addition of an aqueous solution of sodium hydroxide (120 g/l), whereby colloid of zinc hydroxide was formed, and the colloid formed was attached to, and fixed on, the cotton wool to produce deodorizing fibers. The fibers obtained were shaped into a sheet by using a sheet machine and dried to give a sheet of ca. 410 $g/m^2$.

The quantity of zinc hydroxide fixed was 3.2 W/W% (reduced to zinc and based on the weight of the cotton wool), and the rate(%) of zinc fixed was 80 W/W%, based on the weight of zinc added.

EP 0 379 581 B1

5 g of the sheet was dipped with stirring in 50 ml of diluted aqueous ammonia solution (concentration: 2,000 ppm). After it had been allowed to stand for 1 hour, it was tried to smell the odor of ammonia of the aqueous solution, but no ammonia odor was detected.

Example 22

1,000 g of rayon (cellulose fiber) was added to 20 liters of water and disaggregated to the state of slurry by using a disaggregator, and then an aqueous $SO_2$ solution was added thereto up to a pH of 3.0. Subsequently, an aqueous solution of zinc chloride (200 g/l) was added thereto up to a concentration of 4 W/W% (reduced to zinc and based on the weight of the rayon).

Then, an aqueous solution of sodium hydroxide (120 g/l) was added thereto to adjust its pH to 8, whereby colloid of zinc hydroxide was formed, and the colloid formed was attached to, and fixed on, the rayon fibers to produce deodorizing fibers.

The fibers obtained were shaped into a sheet by using a sheet machine and dried to give a sheet of ca. 410 g/m$^2$.

The quantity of zinc fixed was 2.8 W/W% (reduced to zinc and based on the weight of rayon), and the rate(%) of zinc fixed was 70 W/W%, based on the weight of zinc added.

5 g of the sheet was dipped with stirring in 50 ml of diluted aqueous $H_2S$ solution (concentration: 4,000 ppm). After it had been allowed to stand for 1 hour, it was tried to smell the odor of $H_2S$ of the aqueous solution, but no $H_2S$ odor was detected.

Industrial Availability

Copper hydroxide and/or zinc hydroxide can be fixed on cellulose fibers by attaching copper hydroxide and/or zinc hydroxide in a colloidal state onto cellulose fibers in accordance with the process of the present invention. Copper hydroxide and/or zinc hydroxide so fixed are capable of acting on, and exhibiting excellent deodorizing capability for, malodorous gaseous substances, such as hydrogen sulfide, ammonia, methyl mercaptan, etc., in particular, ammonia and hydrogen sulfide, or for ammonia, hydrogen sulfide, etc. dissolved in water.

The deodorizing fibers can be an excellent deodorizer as they are. The fibers, since they are in fibrous form, are also excellent in their processability and can be shaped into a product of any desired shape, including sheets or the like. They are therefore usable as a deodorizing material and can be applied to various uses in the field of deodorization.

Table 1

|  | pH | Amount Fixed (W/W%) | Fixed Rate (%) |
|---|---|---|---|
| Example 1 | 5.0 | 0.2 | 7 |
| Example 2 | 6.0 | 2.0 | 67 |
| Example 3 | 9.5 | 2.5 | 83 |
| Example 4 | 6.5 | 0.2 | 7 |
| Example 5 | 8.0 | 1.8 | 60 |
| Example 6 | 9.5 | 2.3 | 77 |

9

EP 0 379 581 B1

Table 2

| | Cu or Zn components | | | H$_2$S Gas | | | | NH$_3$ Gas | | | |
|---|---|---|---|---|---|---|---|---|---|---|---|
| | Amount Added | Amount Fixed (W/W%) | Fixed Rate (%) | Immediately After Start | Time Lapsed | | | Immediately After Start | Time Lapsed | | |
| | | | | | 10 Min. | 30 Min. | 60 Min. | | 10 Min. | 30 Min. | 60 Min. |
| Example 7 | 0.05 | 0.04 | 80 | 62 | 35 | 15 | 0 | 22 | 21 | 12 | 10 |
| Example 8 | 0.5 | 0.4 | 80 | 45 | 4 | 0 | | 20 | 12 | 8 | 3 |
| Example 9 | 2 | 1.8 | 90 | 12 | 3 | 0 | | 10 | 1 | 0 | |
| Example 10 | 2 | 1.5 | 75 | 22 | 6 | 0 | | 20 | 10 | 5 | 5 |
| Example 11 | 6 | 3.8 | 63 | 30 | 2 | 0 | | 21 | 7 | 2 | 1 |
| Example 12 | Cu 2 Zn 2 | Cu 1.8 Zn 1.3 | 90 65 | 30 | 2 | 0 | | 19 | 7 | 0 | |
| Example 13 | 4 | 4.9 | 79 | 29 | 2 | 0 | | 19 | 5 | 0 | 9 |
| Example 14 | 2 | 1.9 | 95 | 31 | 8 | 0 | | 30 | 12 | 10 | 11 |
| Example 15 | 6 | 3.8 | 63 | 28 | 2 | 0 | | 20 | 8 | 0 | |
| Example 16 | 4 | 2.9 | 72 | 10 | 0 | | | 60 | 35 | 20 | 35 |
| Example 17 | 4 | 2.7 | 68 | 6 | 0 | | | 21 | 17 | 7 | 9 |
| Example 18 | 4 | 2.7 | 68 | 31 | 3 | 0 | | 60 | 28 | 10 | 21 |

Table 3

| | Cu Component | | | Methyl Mercaptan Gas | | | |
|---|---|---|---|---|---|---|---|
| | Amount Added | Amount Fixed (W/W%) | Fixed Rate (%) | Immediately After Start | Time Lapsed 10 Min. | 30 Min. | 60 Min. |
| Example 7 | 0.05 | 0.04 | 80 | 30 | 26 | 20 | 10 |
| Example 8 | 0.5 | 0.4 | 80 | 25 | 25 | 15 | 10 |
| Example 9 | 2 | 1.8 | 90 | 10 | 5 | 5 | 5 |

**Claims**

1. A process for producing deodorizing fibers which is characterized in that copper hydroxide and/or zinc hydroxide in a colloidal solution is adhered to cellulose fibers and fixed thereon.

2. A process for producing deodorizing fibers as claimed in claim 1, wherein an alkaline substance is added to an aqueous solution of a water-soluble copper compound and/or a water-soluble zinc compound in which cellulose fibers are dispersed, so as to form a colloidal solution of copper hydroxide and/or zinc hydroxide and to fix the copper hydroxide and/or zinc hydroxide on said cellulose fibers through contact between them.

3. A process for producing deodorizing fibers as claimed in claim 1, wherein an alkaline substance is added to an aqueous solution of a water-soluble copper compound and/or a water-soluble zinc compound to form a colloidal solution of copper hydroxide and/or zinc hydroxide, and then cellulose fibers are charged and dispersed into the colloidal solution to fix the copper hydroxide and/or zinc hydroxide on the fibers through contact between them.

4. A process for producing deodorizing fibers as claimed in claim 1, wherein said cellulose fibes are treated with an acid before being dispersed into said colloidal solution of a water-soluble copper compound and/or a water-soluble zinc compound.

5. A process for producing deodorizing fibers as claimed in claim 1, wherein said cellulose fibers are dipped in an aqueous solution of chitosan before being dispersed into said colloidal solution of a water-soluble copper compound and/or zinc compound.

6. Deodorizing fibers consisting of cellulose fibers obtained by means of contact of the fibers with copper hydroxide and/or zinc hydroxide in a colloidal solution.

7. Deodorizing fibers as claimed in claim 6, wherein said copper hydroxide and/or zinc hydroxide is fixed on said cellulose fibers via a layer of chitosan.

8. Cellulose fibers as claimed in claim 6, wherein said cellulose fibers are pulp fibers.

9. A deodorizing material which comprises cellulose fibers obtained by contact of the fibers with copper hydroxide and/or zinc hydroxide in a colloidal solution.

10. A deodorizing material as claimed in claim 9, wherein said material is in the form of a sheet.

**Patentansprüche**

1. Verfahren zur Herstellung von deodorierenden Fasern, dadurch gekennzeichnet, daß Kupferhydroxid und/oder Zinkhydroxid in einer kolloidalen Lösung an Zellulosefasern haftet und an diesen befestigt wird.

2. Verfahren zur Herstellung von deodorierenden Fasern nach Anspruch 1, wobei eine alkalische Substanz einer mit Zellulosefasern versetzten wässerigen Lösung aus einer wasserlöslichen Kupferverbindung und/oder einer wasserlöslichen Zinkverbindung zugesetzt wird, zur Bildung einer kolloidalen Lösung aus Kupferhydroxid und/oder Zinkhydroxid und zur Fixierung des Kupferhydroxids und/oder Zinkhydroxids an den genannten Zellulosefasern durch Kontakt zwischen diesen.

3. Verfahren zur Herstellung von deodorierenden Fasern nach Anspruch 1, wobei eine alkalische Substanz einer wässerigen Lösung aus einer wasserlöslichen Kupferverbindung und/oder einer wasserlöslichen Zinkverbindung zugesetzt wird zur Bildung einer kolloidalen Lösung aus Kupferhydroxid und/oder Zinkhydroxid und anschließend Zellulosefasern in die kolloidale Lösung eingebracht und dispergiert werden zur Fixierung des Kupferhydroxids und/oder Zinkhydroxids an den Fasern durch Kontakt zwischen diesen.

4. Verfahren zur Herstellung von deodorierenden Fasern nach Anspruch 1, wobei die genannten Zellulosefasern mit einer Säure behandelt werden bevor sie in der genannten kolloidaale Lösung aus einer wasserlöslichen Kupferverbindung und/oder einer wasserlöslichen Zinkverbindung dispergiert werden.

5. Verfahren zur Herstellung von deodorierenden Fasern nach Anspruch 1, wobei die genannten Fasern in eine wässerige Lösung aus Chitosan eingetaucht werden bevor sie in der kolloidalen Lösung aus einer wasserlöslichen Kupferverbindung und/oder Zinkverbindung dispergiert werden.

6. Deodorierende Fasern bestehend aus Zellulosefasern, die mittels des Kontaktes der Fasern mit Kupferhydroxid und/oder Zinkhydroxid in einer kolloidalen Lösung erhalten wurden.

7. Deodorierende Fasern nach Anspruch 6, wobei das genannte Kupferhydroxid und/oder Zinkhydroxid mittels einer Schicht aus Chitosan an den genannten Zellulosefasern befestigt wird.

8. Zellulosefasern nach Anspruch 6, wobei die genannten Fasern Pulpefasern sind.

9. Deodorierendes Material, das Zellulosefasern aufweist, die durch Kontakt der Fasern mit Kupferhydroxid und/oder Zinkhydroxid in einer kolloidalen Lösung erhalten wurden.

**10.** Deodorierendes Material nach Anspruch 9, wobei das genannte Material die Form eines Blattes aufweist.

**Revendications**

**1.** Procédé pour produire des fibres déodorisantes qui est caractérisé en ce que de l'hydroxyde de cuivre et/ou de l'hydroxyde de zinc dans une solution colloïdale est collé à des fibres de cellulose et fixé sur celles-ci.

**2.** Procédé pour produire des fibres déodorisantes selon la revendication 1 dans lequel une substance alcaline est ajoutée à une solution aqueuse d'un composé de cuivre soluble dans l'eau et/ou d'un composé de zinc soluble dans l'eau dans laquelle des fibres de cellulose sont dispersées de manière à former une solution colloïdale d'hydroxyde de cuivre et/ou d'hydroxyde de zinc et à fixer l'hydroxyde de cuivre et/ou l'hydroxyde de zinc sur les fibres de cellulose mentionnées par contact entre eux.

**3.** Procédé pour produire des fibres déodorisantes selon la revendication 1 dans lequel une substance alcaline est ajoutée à une solution aqueuse d' un composé de cuivre soluble dans l'eau et/ou d'un composé de zinc soluble dans l'eau pour former une solution colloïdale d'hydroxyde de cuivre et/ou d'hydroxyde de zinc, des fibres de cellulose étant ensuite chargées et dispersées dans la solution colloïdale pour fixer l'hydroxyde de cuivre et/ou l'hydroxyde de zinc sur les fibres par contact entre eux.

**4.** Procédé pour produire des fibres déodorisantes selon la revendication 1 dans lequel les fibres de cellulose mentionnées sont traitées avec un acide avant d'être dispersées dans la solution colloïdale mentionnée d'un composé de cuivre soluble dans l'eau et/ou d'un composé de zinc soluble dans l'eau.

**5.** Procédé pour produire des fibres déodorisantes selon la revendication 1 dans lequel les fibres de cellulose mentionnées sont trempées dans une solution aqueuse de chitosane avant d'être dispersées dans la solution colloïdale mentionnée d'un composé de cuivre et/ou d'un composé de zinc soluble dans l'eau.

**6.** Fibres déodorisantes consistant en des fibres de cellulose obtenues au moyen du contact des fibres avec de l'hydroxyde de cuivre et/ou de l'hydroxyde de zinc dans une solution colloïdale.

**7.** Fibres déodorisantes selon la revendication 6 dans lesquelles l'hydroxyde de cuivre et/ou l'hydroxyde de zinc mentionné est fixé sur les fibres de cellulose mentionnées par une couche de chitosane.

**8.** Fibres de cellulose selon la revendication 6 dans lesquelles les fibres de cellulose mentionnées sont des fibres à pulpe.

**9.** Matière déodorisante qui comprend des fibres de cellulose obtenues par contact des fibres avec de l'hydroxyde de cuivre et/ou de l'hydroxyde de zinc dans une solution colloïdale.

**10.** Matière déodorisante selon la revendication 9 dans laquelle ladite matière est en forme de feuille.